Europäisches Patentamt

European Patent Office

Office européen des brevets

(19)

(11) Publication number: **0 239 289 B1**

# EUROPEAN PATENT SPECIFICATION

(12)

(45) Date of publication of patent specification: **27.02.91**

(51) Int. Cl.⁵ **A61K 37/54,** //(A61K37/54, 31:51)

(21) Application number: **87302133.1**

(22) Date of filing: **12.03.87**

(54) **Novel pharmaceutical combinations.**

(30) Priority: **13.03.86 GB 8606283**
**03.10.86 GB 8623835**
**29.01.87 GB 8701976**

(43) Date of publication of application:
**30.09.87 Bulletin 87/40**

(45) Publication of the grant of the patent:
**27.02.91 Bulletin 91/09**

(84) Designated Contracting States:
**BE CH DE FR GB IT LI LU NL SE**

(56) References cited:
**WO-A-86/01104**
**WO-A-86/01110**

(73) Proprietor: **THE WELLCOME FOUNDATION LIMITED**
**Unicorn House 160 Euston Road**
**London NW1 2BP(GB)**

(72) Inventor: **Namm, Donald H.**
**1313 Ravenhurst Drive**
**Raleigh, NC 27609(US)**
Inventor: **Spector, Thomas**
**Route 7 Box 59**
**Durham, NC 27707(US)**
Inventor: **Berger, Henry, Jr.**
**610 Normandy Street**
**Cary, NC 27511(US)**
Inventor: **Frangakis, Crist J.**
**600 Constitution Drive**
**Durham, NC 27705(US)**

(74) Representative: **Stott, Michael John et al**
**The Wellcome Research Laboratories Group**
**Patents & Agreements Langley Court**
**Beckenham Kent BR3 3BS(GB)**

## Description

The present invention relates to a synergistic combination of tissue plasminogen activator and oxypurinol, or a pharmaceutically acceptable salt thereof, to pharmaceutical formulations containing them, and to their use in human and veterinary medicine.

There exists a dynamic equilibrium between the enzyme system capable of forming blood clots, the coagulation system, and the enzyme system capable of dissolving blood clots, the fibrinolytic system, which maintains an intact patent vascular bed. To limit loss of blood from injury, blood clots are formed in the injured vessel. After natural repair of the injury, the superfluous blood clots are dissolved through operation of the fibrinolytic system. Occasionally, blood clots form without traumatic injury and may lodge in major blood vessels resulting in a partial or even total obstruction to blood flow. When this occurs in the heart, lung or brain, the result may be a myocardial infarction, pulmonary embolism or stroke. These conditions combined are the leading cause of morbidity and mortality in the industrialised nations.

Blood clots consist of a fibrous network that is capable of dissolution by the proteolytic enzyme plasmin. The enzyme is derived from the inactive proenzyme, plasminogen, a component of blood plasma, by the action of a plasminogen activator. There are two immunologically distinct mammalian plasminogen activators. Intrinsic plasminogen activator, also known as urokinase, is an enzyme produced by the kidney and can be isolated from urine. It can also be prepared from a number of tissue culture sources. Extrinsic plasminogen activator, also known as vascular plasminogen activator and as tissue plasminogen activator (t-PA), can be isolated from many tissue homogenates (notably human uterus), the vascular cell wall and from some cell cultures. In addition to these two kinds of plasminogen activator, there is also a bacterial product, streptokinase, prepared from beta-haemolytic streptococci. A major drawback with both urokinase and streptokinase is that they are active throughout the circulation and not just at the site of the blood clot. They can, for example, destroy other blood proteins, such as fibrinogen, prothrombin, factor V and factor VIII so reducing blood clotting ability and increasing the risk of haemorrhage. In contrast, the biological activity of t-PA is dependent on the presence of fibrin to which it binds and where it is activated. Maximum activity is thus developed only at the site of a blood clot, i.e. in the presence of the fibrin network to be dissolved, and this greatly avoids the risk of haemorrhage.

The interruption of blood flow in a vessel generally leads to the onset of an ischaemic event. In this condition the tissue is deprived of oxygen and becomes jeopardized, a state in which the tissue is injured but still potentially viable. If however the condition is maintained for a period of, say, three of more hours, the tissue becomes necrotic and, once in this state, cannot be recovered. It is therefore important that reperfusion, i.e. the restoration of blood flow, takes place as soon as possible to salvage the tissue before it becomes permanently damaged. Ironically, reperfusion itself, even if carried out before the tissues becomes necrotic, results in a complex group of phenomena, including the putative formation of the superoxide radical, that have a deleterious effect on hypoxic tissue. Consequently, reperfusion can lead only to the partial recovery of jeopardized tissue, the remainder being permanently damaged by the occurrence of one or more of these phenomena.

Under normal conditions xanthine and hypoxanthine are present in low concentrations in muscle cells and are enzymatically converted to uric acid by xanthine dehydrogenase which uses NAD (nicotinamide-adenine dinucleotide) as an electron acceptor. However in an ischaemic condition the concentrations of xanthine and hypoxanthine generally increase by an order of magnitude or more. It has been proposed that if reperfusion then takes place, the enzyme, xanthine dehydrogenase, will itself be converted to xanthine oxidase. The production of xanthine oxidase will in turn metabolise xanthine and hypoxanthine to uric acid with oxygen being used as the electron acceptor instead of NAD. Superoxide radicals will thus be produced as a by-product ( The New England Journal of Medicine , 1985, 312(3 ), 159-163).

It has now been found that a combination of t-PA and oxypurinol, or a pharmaceutically acceptable salt thereof, inhibits the damage to jeopardized tissue during reperfusion. The combination of t-PA and oxypurinol has been found to provide a significantly potentiated level of inhibition compared with that provided by t-PA or oxypurinol per se . Accordingly the present invention provides a combination of t-PA and oxypurinol, or a pharmaceutically acceptable salt thereof. The present invention affords a particularly convenient means for both the removal of a blood clot and for the inhibition of damage to jeopardized tissue during subsequent reperfusion. Thus, the administration of t-PA and oxypurinol results firstly in the removal of the blood clot through the known thrombolytic action of t-PA and then in the inhibition of tissue damage through the combined action of t-PA and oxypurinol. Although the present invention may be used to protect any jeopardized tissue, it is particularly useful in inhibiting damage to jeopardized myocardial tissue.

The t-PA of use with the present invention may be any bioactive protein substantially corresponding to mammalian, and especially human, t-PA and includes forms with and without glycosylation. It may be one- or two-chain t-PA, or a mixture thereof, as described in EP-A-112 122, and, in the case of fully glycosylated human t-PA, has an apparent molecular weight on polyacrylamide gel of about 70,000 and an isoelectric point of between 7.5 and 8.0. Preferably the t-PA has a specific activity of about 500,000 IU/mg (International Units/mg, the International Unit being a unit of activity as defined by WHO, National Institute for Biological Standards and Control, Holly Hill, Hampstead, London, NW3 6RB, U.K.).

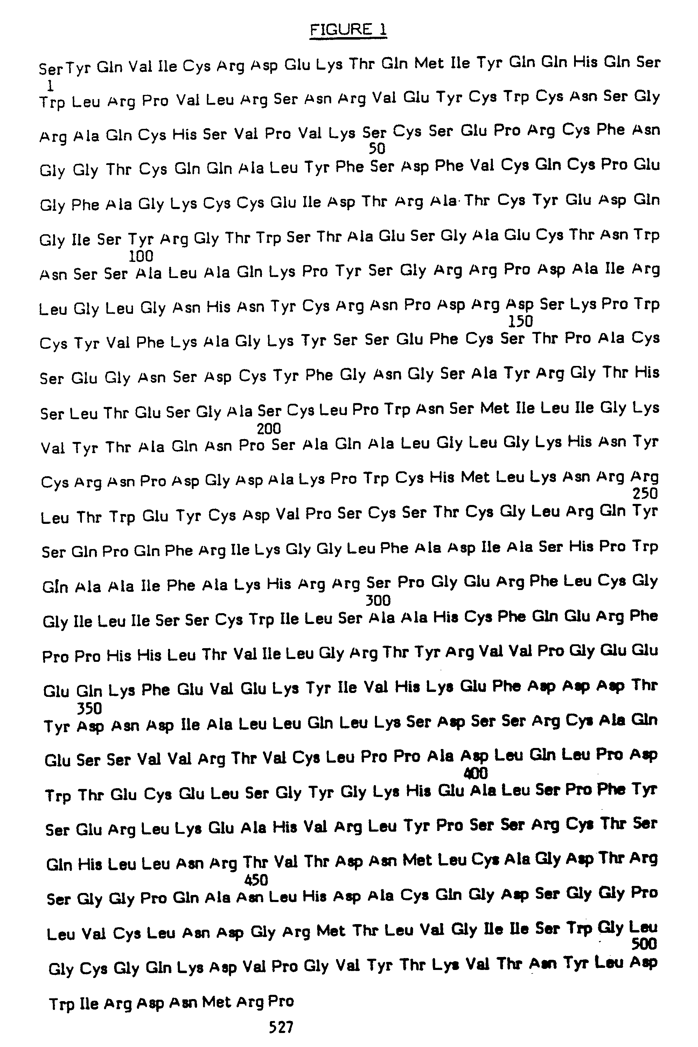

The amino acid sequence of t-PA preferably substantially corresponds to that set forth in Figure 1. The sequence is thus identical to that in Figure 1 or contains one or more amino acid deletions, substitutions, insertions, inversions or additions of allelic origin or otherwise, the resulting sequence having at least 80%, and preferably 90%, homology with the sequence in Figure 1 and retaining essentially the same biological and immunological properties of the protein. In particular, the sequence is identical to that in Figure 1 or has the same sequence but with the amino acid in the 245th position from the serine N-terminus being valine instead of methionine, either sequence optionally being without any of the first three amino acids or optionally having an additional polypeptide N-terminal presequence of Gly-Ala-Arg.

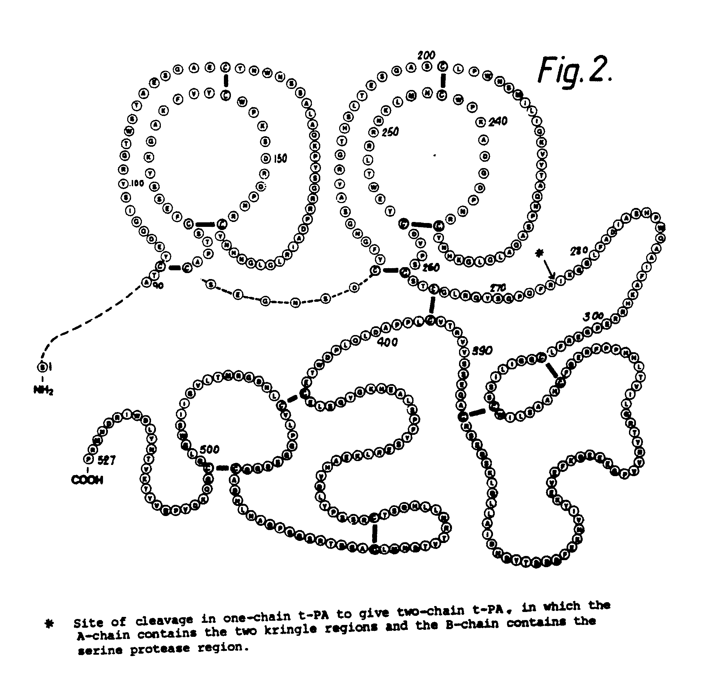

The amino acid sequence set forth in Figure 1 has thirty-five cysteine residues and thus the potential for forming seventeen disulphide bridges. Based on analogy with other proteins whose structure has been determined in more detail, the postulated structure for the sequence (arising from disulphide bond formation) between the amino acid in the 90th position and the proline G-terminus is set forth in Figure 2. The structure of the N-terminal region is less certain although some proposals have been put forward (Progress in Fibrinolysis, 1983 6 , 269-273; and Proc. Natl. Acad. Sci. , 1984, 81 , 5355-5359). The most important features of the structure of t-PA are the two kringle regions (between the 92nd and the 173rd amino acids and between the 180th and 261st amino acids), which are responsible for the binding of the protein to fibrin, and the serine protease region, which comprises the major part of the B-chain and which is responsible for the activation of plasminogen. The amino acids of special significance in serine proteases are the catalytic triad, His/Asp/Ser. In t-PA these occur at the 322nd, the 371st and the 463rd positions. The disulphide bridge between the 264th and 395th cysteine amino acid residues is also important in that it holds together the A- and the B-chains in the two-chain form of t-PA.

In Figures 1 and 2, the conventional one and three letter codes have been employed for the amino acid residues as follows:

| Asp | D | Aspartic acid | Ile | I | Isoleucine | Met | M | Methionine |
|-----|---|---------------|-----|---|------------|-----|---|------------|
| Thr | T | Threonine | Leu | L | Leucine | Asn | N | Asparagine |
| Ser | S | Serine | Tyr | Y | Tyrosine | | | |
| Glu | E | Glutamic acid | Phe | F | Phenylalanine | | | |
| Pro | P | Proline | His | H | Histidine | | | |
| Gly | G | Glycine | Lys | K | Lysine | | | |
| Ala | A | Alanine | Arg | R | Arginine | | | |
| Cys | C | Cysteine | Trp | W | Tryptophan | | | |
| Val | V | Valine | Gln | Q | Glutamine | | | |

The t-PA may be obtained by any of the procedures described or known in the art. For example, it may be obtained from a normal or neoplastic cell line of the kind described in Biochimic a et Biophysica Acta , 1979, 580 , 140-153; EP-A-41 766 or EP-A-113 319. It is preferred, however, that t-PA is obtained from a cultured transformed or transfected cell line derived using recombinant DNA technology as described in, for example, EP-A-93 619; EP-A-117 059; EP-A-117 060; EP-A-173 552; EP-A-174 835; EP-A-178 105; WO 86/01538; WO 86/05514; or WO 86/05807. It is particularly preferred that Chinese hamster ovary (CHO) cells are used for the production of t-PA and are derived in the manner as described in Molecular and Cellular Biology , 1985, 5(7) , 1750-1759. In this way, the cloned gene is cotransfected with the gene encoding dihydrofolate reductase (dhfr) into dhfr⁻ CHO cells. Transformants expressing dhfr are selected on media lacking nucleosides and are exposed to increasing concentrations of methotrexate. The dhfr and t-PA genes are thus coamplified leading to a stable cell line capable of expressing high levels of t-PA.

The t-PA is, preferably, purified using any of the procedures described or known in the art, such as the procedures described in Biochimica et Biophysica Acta , 1979, 580 , 140-153; J . Biol . Chem .,1979, 254-(6) , 1998-2003; ibid , 1981, 256(13) , 7035-7041; Eur . J . Biochem ., 1983, 132 , 681-686; EP-A-41 766;

EP-A-113 319; or GB-A-2 122 219.

Oxypurinol, otherwise known as alloxanthine, has the name, 4,6-dihydroxypyrazolo(3,4-d)pyrimidine, and is the major metabolite of allopurinol, which is the subject of US patent 3 624 205. It may be prepared by any suitable synthetic procedure described or known in the art and is also available commercially from Sigma Chemical Co., St. Louis, Missouri, U.S.A.

Examples of pharmaceutically acceptable salts of oxypurinol include alkali and alkaline earth metal salts. Particularly preferred is the sodium salt.

In using t-PA and oxypurinol, or a pharmaceutically acceptable salt thereof, in the manner of the present invention, it is preferred to employ them in the form of a pharmaceutical formulation. The active ingredients may be employed in separate formulations or in a single combined formulation although in the latter formulation both active ingredients must of course be stable and mutually compatible in the particular formulation employed. The present invention also therefore provides a pharmaceutical formulation, which comprises t-PA and oxypurinol, or a pharmaceutical acceptable salt thereof, and a pharmaceutically acceptable carrier.

Generally, t-PA and oxypurinol, or a pharmaceutically acceptable salt thereof, will be administered by the intravascular route, whether by infusion or by bolus injection, and thus a parenteral formulation is required. It is preferred to present a lyophilised formulation to the physician or veterinarian because of the significant transportation and storage advantages that it affords. The physician or veterinarian may then reconstitute the lyophilised formulation in an appropriate amount of solvent as and when required.

Parenteral and lyophilised pharmaceutical formulations containing t-PA are known in the art. Examples of such art include EP-A-41 766; EP-A-93 619; EP-A-112 122; EP-A-113 319; EP-A-123 304; EP-A-143 081; EP-A-156 169; WO 86/01104; Japanese patent publication 57-120523 (application 56-6936) and Japanese patent publication 58-65218 (application 56-163145). Additional examples include GB-A-2 176 702 and GB-A-2 176 703.

Parenteral pharmaceutical formulations containing oxypurinol include aqueous and non-aqueous sterile solutions and suspensions which may additionally contain an antioxidant, buffer, bacteriostat, isotonic agent or other ingredient to promote bioavailability. Lyophilised pharmaceutical formulations of oxypurinol may be prepared from sterilised solutions by freeze-drying in conventional manner.

Parenteral and lyophilised formulations containing t-PA and oxypurinol together in a single, combined formulation may be prepared in a similar manner to the preparation of formulations suitable for t-PA or oxypurinol per se . As mentioned before, however, the stability and mutual compatibility of the active ingredients in a particular formulation will need to be taken into account and the formulation adapted accordingly.

Intravascular infusions are normally carried out with the parenteral solution contained within an infusion bag or bottle or within an electrically operated infusion syringe. The solution may be delivered from the infusion bag or bottle to the patient by gravity feed or by the use of an infusion pump. The use of gravity feed infusion systems dose not afford sufficient control over the rate of administration of the parenteral solution and, therefore, the use of an infusion pump is preferred especially with solutions containing relatively high concentrations of active ingredients. More preferred, however, is the use of an electrically operated infusion syringe which offers even greater control over the rate of administration.

The present invention also provides a synergistic combination of t-PA and oxypurinol, or a pharmaceutically acceptable salt thereof, for use in human and veterinary medicine, especially for use in inhibiting damage to jeopardized tissue during reperfusion in a mammal.

The present invention is particularly advantageous in inhibiting damage to jeopardized tissue arising from the occurrence of a blood clot in that, as mentioned previously, both the removal of the blood clot and the protection of the jeopardized tissue can be achieved.

In using t-PA and oxypurinol, or a pharmaceutically acceptable salt thereof, in the manner of the present invention, the active ingredients may be administered concurrently or sequentially as separate formulations or as a single combined formulation. If there is sequential administration, it is preferred that the oxypurinol be administered first by, for example, intravenous injection and then followed by a continuous intravascular infusion of t-PA. In any event the delay in administering t-PA should not be such as to lose the benefit of a potentiated effect of the combination of the two components in vivo in inhibiting tissue damage.

An effective amount of t-PA and oxypurinol to inhibit damage to jeopardized tissue during reperfusion will of course depend upon a number of factors including, for example, the age and weight of the mammal, the precise condition requiring treatment and its severity, the route of administration, and will ultimately be at the discretion of the attendant physician or veterinarian. An effective dose, however, in the case of t-PA is likely to be in the range from 150,000 to 1,000,000 IU/kg body weight of patient per hour, preferably in the range from 300,000 to 500,000 IU/kg per hour, and in the case of oxypurinol will be in the range from 0.5 to 10 mg/kg body weight of patient per day, preferably in the range from 2.5 to 5 mg/kg per day.

The following examples are provided in illustration of the present invention.

Example 1:

Preparation of Oxypurinol

To pyrazole-3,4-dicarboxylic acid (7.5g; Chem. Ber ., 1899, 32 , 2292 et seq .) there was added thionyl chloride (150ml). The mixture was refluxed for ten hours. The thionyl chloride was removed in vacuo and the resulting powdery residue added, in portions over 1 hour, to a cold, stirred solution of t -butanol which had previously been saturated with ammonia at 0°C. The mixture was allowed to stand for five hours. The precipitate was removed and boiled with concentrated ammonium hydroxide solution (100ml) for 1 hour. The solution was allowed to evaporate to dryness on a steam bath, and the residue crystallized from boiling water. The compound (pyrazole-3,4-dicarboxamide) thus obtained formed colourless plates and melted with decomposition at 327°C.

To a cold solution of 0.4 M sodium hypochlorite solution (16.6ml) there was added pyrazole-3,4-dicarboxamide (500mg). The reaction mixture turned pink and then faintly yellow. After standing at 0°C for one hour, the mixture was acidified to pH 3 with 2 M(N) hydrochloric acid, and the flocculent precipitate removed. The compound was recrystallized from boiling water to give colourless needles in rosettes of oxypurinol.

Example 2:

Preparation of Injectable Formulation of Oxypurinol

| Ingredients | mg/vial |
|---|---|
| Oxypurinol | 150.0 |
| Sodium Hydroxide, IN | qs to pH 12.5 |
| Water for Injection | qs to 15 mL |

The oxypurinol was added to a solution of sodium hydroxide in 10 mL of water. The pH of the solution was adjusted to 12.5 using sodium hydroxide (IN). After adding additional water, the solution was filtered, sterilised and dispensed into sterilised vials. The solution was lyophilized to form a sterile, dry cake, and the vial sealed under sterile conditions. The solution is reconstituted with water immediately prior to injection.

Example 3:

Preparation of Injectable Formulation of t-PA

A lyophilised injectable formulation of t-PA was prepared substantially as described in GB-A-2 176 702.

Example 4:

Evidence of the Synergistic Effect of t-PA and Oxypurinol in Protecting Jeopardized Tissue

5

(a) Methodology

Male beagle dogs (10-12kg) were anaesthetized with pentobarbital sodium, intubated, and ventilated with room air via a Harvard respirator. Catheters for infusion and arterial blood pressure measurements were implanted in the left jugular vein and left carotid artery. A thoracotomy was performed at the fourth intercostal space, the heart suspended in a pericardial cradle and the left anterior descending artery (LAD) isolated just below the first major diagonal branch. An electromagnetic flow probe was placed on the LAD. A 90 minute occlusion of the LAD was produced by placing a snare of 1/0 silk sutre distal to the flow probe. Treatment was initiated intravenously fifteen minutes prior to release of this snare occlusion and continued for 45 minutes after release. The thoracotomy was closed, and the animals were allowed to recover from the surgical procedures. The animals were reanaesthetized 24 hours after the occlusion, and the flow in the LAD reassessed. Then the heart was removed for post mortem quantification of infarct size.

Five groups of dogs were evaluated. Group 1 consisted of saline controls. Group 2 was administered 1.5 mg/kg of t-PA, Group 3 was administered 3 mg/kg of t-PA, Group 4 was administered 10 mg/kg of the sodium salt of oxypurinol, and Group 5 was administered both 1.5 mg/kg of t-PA and 10 mg/kg of the sodium salt of oxypurinol. The t-PA was administered as a parenteral formulation which was obtained from the lyophilised formulation of Example 3 by reconstitution in water. Similarly, the oxypurinol was administered as a parenteral formulation which was obtained from the lyophilised formulation of Example 2 by reconstitution in water and back titrating with hydrochloric acid to a pH of 9.3. The t-PA had a specific activity of 440,000 IU/mg.

Myocardial infarct size was quantified by an ex vivo dual reperfusion technique. Cannulas were inserted into the LAD immediately distal to the site of occlusion and into the aorta above the coronary ostia. The LAD coronary bed that was perfused with 1.5% triphenyl tetrazolium hydrochloride (TTC) in 0.02 M potassium phosphate buffer, pH 7.4. The aorta was perfused in a retrograde manner with 0.5% Evans blue dye. Both regions were perfused with their respective stains at a constant pressure of 100 mm mercury for five minutes. The heart was cut into 8 mm slices perpendicular to the apex-base axis. The area of the left ventricle at risk of infarction due to anatomical dependence on the LAD for blood flow is identified by the lack of Evans blue in this region. The region of infarcted myocardium within the area at risk was demarcated by the lack of staining of the tissue when perfused with TTC due to a loss of dehydrogenase enzymes.

The transverse ventricular sections were traced carefully on to clear acrylic overlays to provide a permanent record of infarct morphology and to allow planimetric confirmation of infarct size. Ventricular sections then were trimmed of right ventricular muscle, valvular, and fatty tissue. The total left ventricle area at risk and infarct was separated by careful dissection and weighed. Infarct size was expressed as a percentage of the anatomic area at risk. Statistical comparison of the drug treatment group to the control group was made using a one way analysis of variance (anova) using Bonferroni's method for multiple comparison ( Circulation Research , 1980, 47 , 1-9). A p value of less than 0.05 was taken as the criterion of significance.

(b) Results

**TABLE**

| Treatment | Number of dogs | % Left Ventricle* At Risk | % Area at Risk* Infarcted |
|---|---|---|---|
| Saline | 6 | 33.5 ± 2.6 | 37.3 ± 3.5 |
| t-PA (1.5 mg/kg) | 3 | 37.1 ± 7.2 | 31.4 ± 3.1 |
| t-PA (3 mg/kg) | 3 | 32.2 ± 4.7 | 18.8 ± 5.0 |
| Oxypurinol | 6 | 41.7 ± 3.2 | 19.0 ± 1.7 |
| t-PA + Oxypurinol | 3 | 34.4 ± 0.9 | 5.4 ± 0.8 |

* Data are expressed as means ± standard errors.

The proportion of the left ventrical made ischaemic by mechanical occlusion of the LAD was not significantly different between any of the treatment groups and the control group by ANOVA.

(c) Conclusion

The combination of t-PA and oxypurinol achieved a synergistic effect in inhibiting damage to jeopardized tissue during reperfusion.

**Claims**

1. A synergistic combination of t-PA and oxypurinol, or a pharmaceutically acceptable salt thereof.

2. A combination according to claim 1 wherein the t-PA has the amino acid sequence set forth in Figure 1 or has the same amino acid sequence but with the amino acid in the 245th position from the serine N-terminus being valine instead of methionine, either sequence optionally being without any of the first three amino acids or optionally having an additional polypeptide N-terminal presequence of Gly-Ala-Arg.

3. A combination according to either of the preceding claims, wherein the pharmaceutically acceptable salt of oxypurinol is the sodium salt.

4. A pharmaceutical formulation, which comprises a combination according to any one of the preceding claims and a pharmaceutically acceptable carrier.

5. A combination according to any one of claims 1 to 3 for use in human and veterinary medicine.

6. A combination according to claim 5 for use in inhibiting damage to jeopardized tissue during blood reperfusion in a mammal.

7. A combination according to claim 5 for use in the removal of a blood clot and in inhibiting damage to jeopardized tissue during blood reperfusion.

8. Use of t-PA and oxypurinol, or a pharmaceutically acceptable salt thereof, in the preparation of a medicament for inhibiting damage to jeopardized tissue during blood reperfusion in a mammal.

9. Use of t-PA and oxypurinol, or a pharmaceutically acceptable salt thereof, in the preparation of a medicament for the removal of a blood clot and for inhibiting damage to jeopardized tissue during blood reperfusion in a mammal.


## Revendications

1. Combinaison synergique de t-PA et d'oxypurinol, ou d'un sel pharmaceutiquement acceptable de celui-ci.

2. combinaison suivant la revendication 1, dans laquelle le t-PA a la séquence d'acides aminés indiquée à la Fig. 1 ou a la même séquence d'acides aminés, mais avec de la méthionine au lieu de valine comme acide aminé à la position 245 depuis la sérine N-terminale, l'une ou l'autre séquence étant éventuellement exempte des trois premiers acides aminés ou comprenant éventuellement une préséquence Gly-Ala-Arg N-terminale polypeptidique supplémentaire.

3. Combinaison suivant l'une quelconque des revendications précédentes, dans laquelle le sel pharmaceutiquement acceptable d'oxypurinol est le sel sodique.

4. composition pharmaceutique, qui comprend une combinaison suivant l'une quelconque des revendications précédentes et un excipient pharmaceutiquement acceptable.

5. Combinaison suivant l'une quelconque des revendications 1 à 3, à utiliser en médecine humaine et vétérinaire.

6. Combinaison suivant la revendication 5, à utiliser pour inhiber les dégâts dans des tissus à risque pendant la reperfusion sanguine chez un mammifère.

7. combinaison suivant la revendication 5, utilisée pour éliminer un caillot de sang et inhiber les dégâts dans les tissus à risque pendant la reperfusion sanguine.

8. Utilisation du t-PA et de l'oxypurinol, ou d'un sel pharmaceutiquement acceptable de celui-ci, dans la préparation d'un médicament pour inhiber les dégâts dans les tissus à risque pendant la reperfusion sanguine chez un mammifère.

9. Utilisation du t-PA et de l'oxypurinol, ou d'un sel pharmaceutiquement acceptable de celui-ci, dans la préparation d'un médicament pour éliminer un caillot de sang et inhiber les dégâts dans les tissus à risque pendant la reperfusion sanguine chez un mammifère.


## Ansprüche

1. Synergistische Kombination aus t-PA und Oxypurinol oder einem pharmazeutisch annehmbaren Salz davon.

2. Kombination nach Anspruch 1. worin das t-PA die in Fig. 1 angegebene Aminosäuresequenz aufweist oder die gleiche Aminosäuresequenz aufweist, wobei die Aminosäure an der 245. Stelle von dem Serin-Terminus Valin anstelle von Methionin ist, wobei beide Sequenzen gegebenenfalls ohne irgendeine der ersten drei Aminosäuren vorliegt oder gegebenenfalls die zusätzliche N-terminale Polypeptid-Präsequenz Gly-Ala-Arg aufweisen.

3. Kombination nach einem der vorhergehenden Ansprüche, worin als pharmazeutisch annehmbares Salz von Oxypurinol das Natriumsalz vorliegt.

4. Pharmazeutische Zubereitung enthaltend eine Kombination nach einem der vorhergehenden Ansprüche und einen pharmazeutisch annehmbaren Träger.

8

5. Kombination nach einem der Ansprüche 1 bis 3 zur Verwendung in der Human-und Veterinär-Medizin.

6. Kombination nach Anspruch 5 zur Verwendung bei der Inhibierung von Schäden von Gewebe, welches bei der Blutreperfusion bei Säugern gefährdet ist.

7. Kombination nach Anspruch 5 zur Entfernung von Blutklümpchen und zur Inhibierung von Schäden von während der Blutreperfusion gefährdetem Gewebe.

8. Verwendung von t-PA und Oxypurinol oder eines pharmazeutisch annehmbaren Salzes davon zur Herstellung eines Arzneimittels zur Inhibierung von Schäden an Gewebe, welches während der Blutreperfusion bei Säugern gefährdet ist.

9. Verwendung von t-PA und Oxypurinol oder eines pharmazeutisch annehmbaren Salzes davon zur Herstellung eines Arzneimittels zur Beseitigung von Blutklümpchen und zur Inhibierung von Schaden an Gewebe, welches während der Blutreperfusion bei Säugern gefährdet ist.

9

## FIGURE 1

Ser Tyr Gln Val Ile Cys Arg Asp Glu Lys Thr Gln Met Ile Tyr Gln Gln His Gln Ser
1
Trp Leu Arg Pro Val Leu Arg Ser Asn Arg Val Glu Tyr Cys Trp Cys Asn Ser Gly

Arg Ala Gln Cys His Ser Val Pro Val Lys Ser Cys Ser Glu Pro Arg Cys Phe Asn
50
Gly Gly Thr Cys Gln Gln Ala Leu Tyr Phe Ser Asp Phe Val Cys Gln Cys Pro Glu

Gly Phe Ala Gly Lys Cys Cys Glu Ile Asp Thr Arg Ala Thr Cys Tyr Glu Asp Gln

Gly Ile Ser Tyr Arg Gly Thr Trp Ser Thr Ala Glu Ser Gly Ala Glu Cys Thr Asn Trp
100
Asn Ser Ser Ala Leu Ala Gln Lys Pro Tyr Ser Gly Arg Arg Pro Asp Ala Ile Arg

Leu Gly Leu Gly Asn His Asn Tyr Cys Arg Asn Pro Asp Arg Asp Ser Lys Pro Trp
150
Cys Tyr Val Phe Lys Ala Gly Lys Tyr Ser Ser Glu Phe Cys Ser Thr Pro Ala Cys

Ser Glu Gly Asn Ser Asp Cys Tyr Phe Gly Asn Gly Ser Ala Tyr Arg Gly Thr His

Ser Leu Thr Glu Ser Gly Ala Ser Cys Leu Pro Trp Asn Ser Met Ile Leu Ile Gly Lys
200
Val Tyr Thr Ala Gln Asn Pro Ser Ala Gln Ala Leu Gly Leu Gly Lys His Asn Tyr

Cys Arg Asn Pro Asp Gly Asp Ala Lys Pro Trp Cys His Met Leu Lys Asn Arg Arg
250
Leu Thr Trp Glu Tyr Cys Asp Val Pro Ser Cys Ser Thr Cys Gly Leu Arg Gln Tyr

Ser Gln Pro Gln Phe Arg Ile Lys Gly Gly Leu Phe Ala Asp Ile Ala Ser His Pro Trp

Gln Ala Ala Ile Phe Ala Lys His Arg Arg Ser Pro Gly Glu Arg Phe Leu Cys Gly
300
Gly Ile Leu Ile Ser Ser Cys Trp Ile Leu Ser Ala Ala His Cys Phe Gln Glu Arg Phe

Pro Pro His His Leu Thr Val Ile Leu Gly Arg Thr Tyr Arg Val Val Pro Gly Glu Glu

Glu Gln Lys Phe Glu Val Glu Lys Tyr Ile Val His Lys Glu Phe Asp Asp Asp Thr
350
Tyr Asp Asn Asp Ile Ala Leu Leu Gln Leu Lys Ser Asp Ser Ser Arg Cys Ala Gln

Glu Ser Ser Val Val Arg Thr Val Cys Leu Pro Pro Ala Asp Leu Gln Leu Pro Asp
400
Trp Thr Glu Cys Glu Leu Ser Gly Tyr Gly Lys His Glu Ala Leu Ser Pro Phe Tyr

Ser Glu Arg Leu Lys Glu Ala His Val Arg Leu Tyr Pro Ser Ser Arg Cys Thr Ser

Gln His Leu Leu Asn Arg Thr Val Thr Asp Asn Met Leu Cys Ala Gly Asp Thr Arg
450
Ser Gly Gly Pro Gln Ala Asn Leu His Asp Ala Cys Gln Gly Asp Ser Gly Gly Pro

Leu Val Cys Leu Asn Asp Gly Arg Met Thr Leu Val Gly Ile Ile Ser Trp Gly Leu
500
Gly Cys Gly Gln Lys Asp Val Pro Gly Val Tyr Thr Lys Val Thr Asn Tyr Leu Asp

Trp Ile Arg Asp Asn Met Arg Pro
527

Fig.2.

\* Site of cleavage in one-chain t-PA to give two-chain t-PA, in which the A-chain contains the two kringle regions and the B-chain contains the serine protease region.